# EUROPEAN PATENT SPECIFICATION

(11) **EP 1 511 381 B1**
(45) Date of publication and mention of the grant of the patent: **21.08.2013**
(21) Application number: 03755238.7
(22) Date of filing: 28.03.2003
(51) Int. Cl.: A01N 37/06, A01N 37/02, A01N 33/12, A01N 33/04

(54) **DISINFECTANT WITH ACTIVITY AGAINST HEPATITIS B VIRUS**
DESINFEKTIONSMITTEL MIT WIRKUNG GEGEN HEPATITIS-B-VIRUS
DESINFECTANT AYANT UNE ACTIVITE CONTRE LE VIRUS DE L'HEPATITE B

(30) Priority: 29.05.2002 DE 10223934
(43) Date of publication of application: 09.03.2005
(73) Proprietor: Air Liquide Santé (International), 75341 Paris Cedex 07 (FR); SCHÜLKE & MAYR GMBH, 22851 Norderstedt (DE)
(72) Inventor: GORONCY-BERMES, Peter, 22145 Hamburg (DE); MOHR, Michael, 24568 Kaltenkirchen (DE)
(74) Representative: Conan, Philippe Claude
(86) International application number: PCT/IB2003/001137
(87) International publication number: WO 2003/099006

(56) References cited:
- EP-A- 0 147 976
- EP-A- 0 339 957
- EP-A- 0 520 785
- EP-A- 1 195 091
- WO-A-98/03066
- US-A- 4 110 504
- US-A- 4 585 795
- US-A- 6 054 139
- DATABASE WPI Section Ch, Week 199650 Derwent Publications Ltd., London, GB; Class B05, AN 1996-502627 XP002244995 & JP 08 259444 A (EARTH SEIYAKU KK), 8 October 1996 (1996-10-08)

## Description

The invention relates to disinfectants and to the use thereof for inactivating hepatitis B virus.

Three groups of substances have been used to date in particular for the inactivation of viruses such as hepatitis B virus using surface (skin, floor) and instrument disinfectants:
- Short-chain organic acids such as formic acid, acetic acid, citric acid. The effect of such monobasic or polybasic acids is disclosed inter alia in EP-A-0 505 763 and AT-A 3823190, see also Hygiene + Medizin 1989, 14, pages 69 et seq., GB-A-2 103 089 and Tierârztliche Umschau 1988, 43, pages 646 et seq. However, a disadvantage which has emerged is that such disinfectants inevitably have a low pH and accordingly have a strong corrosive action especially when used at elevated temperatures for disinfecting instruments.
- Quaternary ammonium compounds.

These have proved to be, especially in disinfectants with a very high alcohol content, e.g. in anhydrous isopropanol / n-propanol or 80% ethanol, effective disinfectants for the hands (see, inter alia, Wallhâuser, Praxis der Sterilisation, Henkel Chemische Bibliothek, 4th edition, 1988, pages 75 et seq.). However, tests by the Applicant have shown that quaternary ammonium compounds are not reliably effective for HBV in solutions with very low alcohol content. On the other hand, disinfectants with high alcohol concentrations are unsuitable inter alia for disinfecting instruments because they attack plastic materials. In addition, disinfectants containing quaternary ammonium compounds are high-foaming, which restricts their use, especially for disinfecting instruments.
- Aldehydes such as formaldehyde, acetaldehyde and glutaraldehyde. Aldehyde-containing products show excellent activity against HBV. However, aldehyde-containing disinfectants have been unwanted for some years because of the harmful effects on human health, especially of formalin.

Accordingly, the object on which the present invention was based was to provide disinfectants which
1. for reasons of cost and for good tolerability on possible contact of the user with the disinfectantare low in active ingredient and well tolerated (non-irritant) for people,
2. are not inevitably strongly acidic and corrosive,
3. have no foaming action,
4. can be formulated with low alcohol content,
5. do not inevitably require the presence of aldehyde,
6. are compatible with other optional ingredients and
7. inactivate hepatitis B virus outstandingly even used in low concentration.

The proposal according to the invention for the inactivation of HBV is the non therapeutic use of a water-based disinfectant which comprises:
a) dioctyldimethylammonium chloride,
b) sodium oleate and
c) a non ionic surfactant,
for disinfecting instruments, by inactivation of Hepatitis B virus.

According to the invention, a mixture of dioctyldimethylammonium chloride with bis(3-amino propyl) octylamine can also be used.
Disinfectants which have proved to be particularly effective comprise from 1 to 40% by weight, preferably 3 to 25% by weight, in particular 5 to 20% by weight, of component a) based on the disinfectant.

In a disinfectant according to the invention, the content of component b) is preferably from 0.05 to 5% by weight, in particular 0.1 to 3% by weight, such as 0.5 to 2% by weight, based on the disinfectant (calculated as free acid RCOOH).

In a particularly preferred embodiment, the disinfectant comprises
a) 5 to 10% by weight of dioctyldimethylammonium chloride,
b) 0.3 to 3% by weight of sodium oleate and
c) 0.2 to 1.0% by weight of nonionic surfactant.

Besides the components which are stipulated as obligatory according to the invention, the disinfectant used according to the invention additionally comprises where appropriate one or more other substances such as short-chain organic acids such as lactic acid, glycolic acid, citric acid, malic acid, succinic acid, tartaric acid, formic acid, acetic acid, propionic acid or salts thereof, corrosion inhibitors, perfume, dye and alcohols. The short-chain organic acids are employed in particular for adjusting the amine formulations to the preferred pH of from 9.0 to 9.5. The content of every single one of the other substances is preferably up to 5% by weight.

A preferred alcohol is isopropanol. It is also possible to employ in addition or in place of isopropanol other alcohols such as ethanol or n-propanol or glycols or aromatic alcohols such as phenoxypropanols, which act as solubilizers to stabilize the concentrate. The alcohols prevent crystallization, improve the low- temperature stability and serve as further active ingredients; their concentration can also be distinctly higher than 5% by weight and be, for example, from 10 to 50% by weight, such as 20 to 40% by weight.

In another particular embodiment, the disinfectant comprises:
a) 10 to 20% by weight, preferably 13 to 17% by weight, of dioctyldimethylammonium chloride,
b) 2 to 7% by weight, of sodium oleate,
c) 1 to 3 % by weight, of nonionic surfactant and
d) 10 to 50% by weight, of alcohol.

In a particularly preferred embodiment, the disinfectant displays as 2% by weight aqueous solution a foaming power of less than 45 ml, preferably less than 42 ml, in particular less than 39 ml or even 37 ml or less, determined by the following method:
1. Foam-free introduction of 30 ml of solution into a 100 ml measuring cylinder at 20 to 25°C,
2. Placing of a stopper on the measuring cylinder,
3. Vigorous shaking ten times and
4. After the end of the shaking immediate determination of the height of the foam, stated as volume of the solution including foam.

The invention further relates to the use of the disinfectant for disinfecting surfaces and instruments. Surfaces are generally disinfected by scouring or wiping methods. Instruments are disinfected by manual insertion of the instruments or by mechanical methods in automatic processors. Typically employed in the said method is a ready-to-use solution which represents an aqueous solution of the disinfectant and comprises from 0.3 to 10% by weight, preferably 0.5 to 5% by weight, in particular 1 to 3 % by weight, e.g. 2% by weight, of the disinfectant.

The thermochemical disinfection of instruments, especially temperature-sensitive instruments such as flexible endoscopes, is carried out in special automatic cleaning and disinfection systems. An example of a program flow in which the disinfectant according to the invention can be employed advantageously in the form of an instrument disinfectant is as follows:
1. Where appropriate precleaning with cold water,
2. Cleaning at 55 to 60°C with a neutral cleaner,
3. Thermochemical disinfection at 55 to 60°C and an exposure time of from 1 to 20 minutes to a disinfectant according to any of Claims 1 to 5,
4. Rinsing with cold water and
5. Drying.

For thermochemical disinfection, the three parameters of concentration, exposure time and temperature are chosen suitably by the skilled person.
In another embodiment, the invention relates to a ready-to-use water solution comprising 0.3 to 10% of a water based disinfectant comprising:
a) 5 to 20% by weight of dioctyldimethylammonium chloride,
b) 0.5 to 2% by weight of sodium oleate and
c) 0.2 to 1.0% by weight of nonionic surfactant,
for use in a method for an external skin treatment of Hepatitis B virus inactivation.

Particularly preferred disinfectants contain the following components in the following quantities:
(i) Surface disinfectant (data in % by weight)

| | preferred | especially |
|---|---|---|
| dioctyldimethylammonium chloride | 5-10 | 6-9 |
| Arom. alcohoi | no | |
| oleic acid, sodium sait | 0.3-3 | 0.8-1.6 |
| Nonionic surfactant | 0.2-1 | 0.3-0.7 |
| Alcohol | no | |
| Amine (pH adjustment) | no | |
| Corrosion inhibitor | yes | |
| Perfume | yes | |
| Dye | yes | |

(ii) Instrument disinfectant (data in percent by weight)

| | preferred | especially |
|---|---|---|
| Quaternary ammonium compound, in particular dioctyldimethylammonium chloride | 10-20 | 13-17 |
| Arom. alcohol | 1-10 | 3-7 |
| Fatty acid, in particular oleic acid, as sodium salt | 1-7 | 3-5 |
| Nonionic surfactant | 1-3 | 1.5-2.5 |
| Alcohol | 10-50 | 20-40 |
| Amine (pH adjustment) | yes | |
| Corrosion inhibitor | yes | |
| Perfume | no | |
| Dye | no | |

The advantages of the invention are evident in particular from the following examples. Unless stated otherwise, all percentage data in the examples are based on weight.

### Examples

The following substances were used:
- Bardac LF: dioctydimethylammonium chloride (50% strength),
- Lonzabac LF: bis(3-aminopropyl)octylamine and
- nonionic surfactant: alkylpolyoxylakylene glycol ether (low-foaming surfactant).

### Formulations based on quaternary ammonium compound

| | 1A (Comparative) | 1B |
|---|---|---|
| Bardac LF | 15% | 15% |
| Sodium oleate | | 1% |
| Sodium citrate | 5% | |
| Nonionic surfactant | 2% | 2% |
| Water | 78% | 82% |

### Formulations based on alkylamine

| | 2A (Comparative) | 2B (Reference example) |
|---|---|---|
| Lonzabac LF | 7.5% | 7.5% |
| Sodium oleate | | 1.5% |
| Nonionic surfactant | 4% | |
| Malic acid | 3.5% | 3.5% |
| Corrosion inhibitor | 0.1% | 0.1% |
| Isopropanol | | 5% |
| Water | 84.9% | 78.4% |

### Investigation of the HBV activity of formulations

For the investigations with the formulations, the destruction of the antigenicity of the surface antigen (hepatitis B surface antigen = HBsAG) was used as indirect marker of hepatitis B activity. The formulations were in each case diluted with sterile double-distilled water to the desired concentrations (1% by weight, 2% by weight and 3% by weight) immediately before the inactivation tests.

The disinfectant tests took place in a suspension test at room temperature, with the volume ratios and the protein loading being carried out in accordance with the guideline of the German health agency (BGA) and the German association for controlling viral diseases (DVV) on the testing of the activity of chemical disinfectants against viruses, see Bundesgesundheitsblatt 25, 1982, pages 397-8. The test mixture consisted of one part of an HBsAg-containing serum (HBsAg and HBeAg pos., DNA polymerase detectable, HBV PCR pos., virus genomes ≥ 10⁸/ml), one part of double-distilled water or one part of a 2% strength serum albumin solution or one part of foetal calf serum (FCS) and eight parts by volume of the formulation to be tested (disinfectant) in 1.25 times the desired concentration. Immediately after the exposure time had elapsed, the test mixture was diluted 1:100 with PBS containing 10% FCS in order to abolish the effect of the disinfectant by dilution. Each mixture was subsequently investigated as duplicate determination of HBsAg in a radioimmunoassay (RIA) (Ausria II, Abbott Lab., North Chicago, I11., USA) and the average radioactivity was calculated.

The following controls were also included. A test mixture containing double-distilled water in place of the disinfectant was investigated for HBsAg after the longest of the tested exposure times. This mixture, which was also carried out with serum albumin and FCS, represented the initial values for calculating the increase in HBsAg. A test mixture without added virus (disinfectant controls) and a test mixture exclusively with the diluent also took place in order to identify in this way, by comparison of the values, a nonspecificity due to disinfectant present (see attached key).

Positive and negative controls from the manufacturer's test kit were also included.

Then, in accordance with the method described by G. Frösner, G. Jentsch, H. Uthermann in Zbl. Bakt. Hyg. I. Abt. Orig. B 176, 1, (1982) "Zerstörung der Antigenität und Beeinflussung der immunochemischen Reaktivität von Antigenen des Hepatitis-B-Virus (HbsAg, HbcAg and HbeAg) durch Desinfektionsmittel im Prüfmodell", complete inactivation of HBsAg was assumed if the radioactivity (cpm) after exposure to the disinfectant was less than 2.1 times the radioactivity (cpm) of the test mixture without added virus. The disinfectant in these test mixtures was mixed with double-distilled water, serum albumin or FCS, and was then diluted 1:100 in PBS with 10% FCS in accordance with the description above.

### Explanation of the mixtures:

### HBsAG controls

I serum + double-distilled water + double-distilled water
II serum + 2% albumin + double-distilled water
III serum + FCS + double-distilled water

### Disinfectant controls

I double-distilled water + double-distilled water + disinfectant
II double-distilled water + 2% albumin + disinfectant
III double-distilled water + FCS + disinfectant

### Diluent control

I 10% FCS in PBS

### Inactivation mixture

I serum + double-distilled water + disinfectant
II serum + 2% albumin + disinfectant
III serum + FCS + disinfectant

### Investigation of the foaming power of formulations

The following tests serve to assess the foaming power of a formulation solution (disinfectant solution).

A 100 ml measuring cylinder (high form with graduation and lettering) with a fitting stopper and a stop-clock are required. For the investigation, 30 ml of the formulation solution to be tested are introduced into the measuring cylinder, avoiding foaming as far as possible (if foam has formed on introduction of the formulation solution, the test is not carried out until the foam has disappeared.) The stopper is then put on. The measuring cylinder is shaken vigorously ten times and then put down, starting the stop-clock simultaneously. The height of the foam in the measuring cylinder (volume of the sample including foam) is then read off after predetermined times. The results of the investigation are recorded, stating the sample temperature.

### Example 1

Formulation 1B was investigated as 1.0% strength, 2.0% strength and 3.0% strength solution in the inactivation tests. The exposure times were 5, 15 and 30 minutes. The results are shown in Tables 1-3 below.

**Table 1: Hepatitis B-inactivating properties of formulation 1B (1.0%) in the antigen assay. The cpm are shown.**

| Exposure time (min) | HBsAg controls | | | Disinfectant controls | | | Inactivation mixture 1B (1.0%) | | |
|---|---|---|---|---|---|---|---|---|---|
| | I | II | III | I | II | III | I | II | III |
| 1 | - | - | - | - | - | - | n.d. | n.d. | n.d. |
| 5 | - | - | - | - | - | - | 1970 | 2256 | 3629 |
| 15 | - | - | - | - | - | - | 1568 | 1781 | 2625 |
| 30 | 7164 | 6609 | 6708 | 125 | 131 | 133 | 1017 | 1560 | 2141 |

| | | | | | | | | | |
|---|---|---|---|---|---|---|---|---|---|
| n.d. = not done Diluent control: 186.0 Lower limit of detection of HBsAg I : 262.5 in the individual mixtures (cut off) II : 275.1 III : 279.3 | | | | | | | | | |

**Table 2: Hepatitis B-inactivating properties of formulation 1B (2.0%) in the antigen assay. The cpm are shown.**

| Exposure time (min) | HBsAg controls | | | Disinfectant controls | | | Inactivation mixture 1B (2.0%) | | |
|---|---|---|---|---|---|---|---|---|---|
| | I | II | III | I | II | III | I | II | III |
| 1 | - | - | - | - | - | - | n.d. | n.d. | n.d. |
| 5 | - | - | - | - | - | - | 348 | 448 | 560 |
| 15 | - | - | - | - | - | - | 205 | 238 | 249 |
| 30 | 7164 | 6609 | 6708 | 117 | 115 | 121 | 101 | 105 | 131 |

| | | | | | | | | | |
|---|---|---|---|---|---|---|---|---|---|
| Diluent control: 186.0 Lower limit of detection of HBsAg I : 245.7 in the individual mixtures (cut off) II : 241.5 III : 254.1 | | | | | | | | | |

**Table 3: Hepatitis B-inactivating properties of formulation 1B (3.0%) in the antigen assay. The cpm are shown.**

| Exposure time (min) | HBsAg controls | | | Disinfectant controls | | | Inactivation mixture 1B (3.0%) | | |
|---|---|---|---|---|---|---|---|---|---|
| | I | II | III | I | II | III | I | II | III |
| 1 | - | - | - | - | - | - | n.d. | n.d. | n.d. |
| 5 | - | - | - | - | - | - | 217 | 339 | 464 |
| 15 | - | - | - | - | - | - | 117 | 127 | 125 |
| 30 | 7164 | 6609 | 6708 | 121 | 110 | 127 | 87 | 99 | 97 |

| | | | | | | | | | |
|---|---|---|---|---|---|---|---|---|---|
| Diluent control: 186.0 Lower limit of detection of HBsAg I : 254.1 in the individual mixtures (cut off) II : 231.0 III : 266.7 | | | | | | | | | |

### Evaluation of example 1

No interference with the detection method by the presence of disinfectant was evident because the results for the disinfectant controls were in the region of the value for the diluent (cpm = 186.0).

Formulation 1B showed a strong effect on the immunological reactivity of HBsAg. However, in the investigation of the 1% strength solution, counts above the lower limit of detection were not seen until after an exposure time of 30 minutes (see Table 1). The 2% strength dilution of the disinfectant in particular showed HBV activity (see Table 2). It can be stated as a result that a 2% strength solution of formulation 1B can be employed for HBV inactivation on use for 15 minutes as surface disinfectant.

According to the Deutsches Ärzteblatt 84, No. 18, page B874 of 30 April 1987, the committee on questions of viral disinfection in human medicine of the German association for the control of viral diseases (DVV) and of the German health agency (BGA) have summed up that all precautionary measures against transmission of hepatitis B are also HIV-preventive.

Exemplary formulation 1B shows that the activity of quaternary ammonium compounds against HBV is increased through use of 1% by weight fatty acid salt (sodium oleate). It was also possible to reduce the total amount of active ingredients.

### Example 2

Formulations 2A and 2B were tested for their HBV activity correspondingly. The results are shown in Table 4 below.

**Table 4: HBV activity of formulations based on quaternary ammonium compound**

| | 2A (Comparative) | | | | 2B (Reference example) | | | |
|---|---|---|---|---|---|---|---|---|
| HBV activity (2% strength in water) | cut off | 15 min | 30 min | 60 min | cut off | 15 min | 30 min | 60 min |
| | 212.2 | 2020 | 1515 | 1261 | 178.5 | 141 | 92 | 96 |

### Example 3

The procedure was as in Examples 1 and 2, but the suspensions were heated to 55°C.

**Table 5: HBV activity of formulations based on alkylamine (at 55°C)**

| | 2A (Comparative) | | | | 2B (Reference example) | | | |
|---|---|---|---|---|---|---|---|---|
| HBV activity (2% strength in water) | cut off | 15 min | 30 min | 60 min | cut off | 15 min | 30 min | 60 min |
| | 333.9 | 7741 | 6798 | 6302 | 312.9 | 593 | 290 | 281 |

It is possible through the use of sodium oleate both to reduce foaming and to improve the action on HBV.

### Example 4 (reduction in foaming)

### Formulations based on quaternary ammonium compound

| | 4A (Comparative) | 4B | 4C (Comparative) |
|---|---|---|---|
| Bardac LF | 15% | 15% | 15% |
| Sodium oleate | | 1% | |
| Sodium citrate | 5% | | |
| Nonionic surfactant | 2% | 2% | 2% |
| Water | 78% | 82% | 83% |

The formulations solutions were 2% strength solutions in tap water at 22°C.

**Table 6: Foaming of various disinfectant solutions**

| | 4A (Comparative) | 4B | 4C (Comparative) |
|---|---|---|---|
| Foaming power, total volume [ml] after 0 s | 37 | 47 | 72 |
| Foaming power, total volume [ml] after 30 s | 33 | 40 | 70 |
| after 1 min | 33 | 38 | 69 |
| after 2 min | 32 | 37 | 67 |
| after 3 min | 32 | 35 | 53 |
| after 4 min | 32 | 35 | 48 |
| after 5 min | 32 | 35 | 45 |

### Result:

Formulation 4A showed foam suppression
Formulation 4B showed foam suppression
Formulation 4C showed no foam suppression

## Claims

1. Non therapeutic use of a water - based disinfectant which comprises:
a) dioctyldimethylammonium chloride,
b) sodium oleate and
c) a non ionic surfactant,
for disinfecting surfaces and instruments, by inactivation of Hepatitis B virus.

2. Non therapeutic use according to Claim 1, **characterized in that** the disinfectant comprises from 1 to 40% by weight, of component a), based on the disinfectant.

3. Non therapeutic use according to any of Claim 1 or 2, **characterized in that** the disinfectant comprises from 0.05 to 5% by weight of component b) calculated as free acid, based on the disinfectant.

4. Non therapeutic use according to any of preceding claims, **characterized in that** the disinfectant comprises:
a) 5 to 10% by weight of dioctyldimethylammonium chloride,
b) 0.3 to 3% by weight of sodium oleate and
c) 0.2 to 1.0% by weight of nonionic surfactant.

5. Non therapeutic use according to Claim 4, **characterized in that** the disinfectant comprises:
a) 10 to 20% by weight of dioctyldimethylammonium chloride,
b) 2 to 7% by weight, of sodium oleate,
c) 1 to 3 % by weight, of nonionic surfactant and
d) 10 to 50% by weight, of alcohol.

6. Non therapeutic use according to any of preceding claims, **characterized in that** the disinfectant is formulated with water to a ready-to-use solution which comprises 0.3 to 10% by weight, of the disinfectant.

7. Method for the thermochemical disinfection of instruments which comprises the following steps:
1. Where appropriate precleaning with cold water,
2. Cleaning at 55 to 60°C with a neutral cleaner,
3. Thermochemical disinfection at 55 to 60°C and an exposure time of from 1 to 20 minutes to a disinfectant according to any of Claims 1 to 5,
4. Rinsing with cold water and
5. Drying.

8. A ready-to-use water solution comprising 0.3 to 10% of a water based disinfectant comprising:
a) 5 to 20% by weight of dioctyldimethylammonium chloride,
b) 0.5 to 2% by weight of sodium oleate and
c) 0.2 to 1.0% by weight of nonionic surfactant,
for use in a method for an external skin treatment of Hepatitis B virus inactivation.

## Patentansprüche

1. Nicht therapeutische Verwendung eines Desinfektionsmittels auf Wasser-Basis, welches umfasst:
a) Dioctyldimethylammoniumchlorid,
b) Natriumoleat und
c) ein nichtionisches grenzflächenaktives Mittel,
zum Desinfizieren von Oberflächen und Instrumenten durch lanktivierung des Hepatitis B Virus.

2. Nicht therapeutische Verwendung gemäß Anspruch 1, **dadurch gekennzeichnet, dass** das Desinfektionsmittel 1 bis 40 Gew.-% an Bestandteil a) auf Basis des Desinfektionsmittels umfasst.

3. Nicht therapeutische Verwendung gemäß Anspruch 1 oder 2, **dadurch gekennzeichnet, dass** das Desinfektionsmittel 0,05 bis 5 Gew.-% an Bestandteil b), berechnet als freie Säure, auf Basis des Desinfektionsmittels umfasst.

4. Nicht therapeutische Verwendung gemäß einem der vorherigen Ansprüche, **dadurch gekennzeichnet, dass** das Desinfektionsmittel umfasst:
a) 5 bis 10 Gew.-% an Dioctyldimethylammoniumchlorid,
b) 0,3 bis 3 Gew.-% an Natriumoleat und
c) 0,2 bis 1,0 Gew.-% an einem nichtionischen grenzflächenaktiven Mittel.

5. Nicht therapeutische Verwendung gemäß Anspruch 4, **dadurch gekennzeichnet, dass** das Desinfektionsmittel umfasst:
a) 10 bis 20 Gew.-% an Dioctyldimethylammoniumchlorid,
b) 2 bis 7 Gew.-% an Natriumoleat,
c) 1 bis 3 Gew.-% an einem nichtionischen grenzflächenaktiven Mittel und
d) 10 bis 50 Gew.-% an Alkohol.

6. Nicht therapeutische Verwendung gemäß einem der vorherigen Ansprüche, **dadurch gekennzeichnet, dass** das Desinfektionsmittel mit Wasser zu einer verwendungsbereiten Lösung formuliert ist, welche 0,3 bis 10 Gew.-% des Desinfektionsmittels umfasst.

7. Verfahren zur thermochemischen Desinfektion von Instrumenten, welches die folgenden Schritte umfasst:
1. falls dienlich, Vorreinigen mit kaltem Wasser,
2. Reinigen bei 55 bis 60°C mit einem neutralen Reinigungsmittel,
3. thermochemische Desinfektion bei 55 bis 60°C und einer Einwirkzeit von 1 bis 20 Minuten eines Desinfektionsmittels gemäß einem der Ansprüche 1 bis 5,
4. Spülen mit kaltem Wasser und
5. Trocknen.

8. Eine verwendungsbereite Wasserlösung, umfassend 0,3 bis 10% eines Desinfektionsmittels auf Wasser-Basis umfassend:
a) 5 bis 20 Gew.-% an Dioctyldimethylammoniumchlorid,
b) 0,5 bis 2 Gew.-% an Natriumoleat und
c) 0,2 bis 1,0 Gew.-% an einem nichtionischen grenzflächenaktiven Mittel,
zur Verwendung in einem Verfahren für eine äußere Hautanwendung zur Hepatitis B Virusinaktivierung.

## Revendications

1. Utilisation non thérapeutique d'un désinfectant à base d'eau qui comprend :
a) du chlorure de dioctyldiméthylammonium,
b) de l'oléate de sodium et
c) un agent tensioactif non ionique,
pour désinfecter des surfaces et des instruments par inactivation du virus de l'hépatite B.

2. Utilisation non thérapeutique selon la revendication 1, **caractérisée en ce que** le désinfectant comprend 1 à 40 % en poids de composant a) sur la base du désinfectant.

3. Utilisation non thérapeutique selon l'une quelconque des revendications 1 ou 2, **caractérisée en ce que** le désinfectant comprend 0,05 à 5 % en poids de composant b) calculés comme acide libre sur la base du désinfectant.

4. Utilisation non thérapeutique selon l'une quelconque des revendications précédentes, **caractérisée en ce que** le désinfectant comprend :
a) 5 à 10 % en poids de chlorure de dioctyldiméthyl-ammonium,
b) 0,3 à 3 % en poids d'oléate de sodium et
c) 0 , 2 à 1 , 0 % en poids d'agent tensioactif non ionique.

5. Utilisation non thérapeutique selon la revendication 4, **caractérisée en ce que** le désinfectant comprend :
a) 10 à 20 % en poids de chlorure de dioctyldiméthyl-ammonium,
b) 2 à 7 % en poids d'oléate de sodium,
c) 1 à 3 % en poids d'agent tensioactif non ionique et
d) 10 à 50 % en poids d'alcool.

6. Utilisation non thérapeutique selon l'une quelconque des revendications précédentes, **caractérisée en ce que** le désinfectant est formulé avec de l'eau pour obtenir une solution prête à l'emploi qui comprend 0,3 à 10 % en poids du désinfectant.

7. Procédé de désinfection thermochimique d'instruments, qui comprend les étapes consistant à :
1. lorsque cela s'avère approprié, pré-nettoyer à l'eau froide,
2. nettoyer à une température de 55 à 60 °C avec un nettoyant neutre,
3. effectuer une désinfection thermochimique à une température de 55 à 60 °C et durant une période d'exposition de 1 à 20 minutes à un désinfectant selon l'une quelconque des revendications 1 à 5,
4. rincer à l'eau froide et
5. sécher.

8. Solution aqueuse prête à l'emploi comprenant 0,3 à 10 % d'un désinfectant à base d'eau, comprenant :
a) 5 à 20 % en poids de chlorure de dioctydiméthyl-ammonium,
b) 0,5 à 2 % en poids d'oléate de sodium et
c) 0,2 à 1,0 % en poids d'agent tensioactif anionique,
pour utilisation dans un procédé de traitement de la peau externe par inactivation du virus de l'hépatite B.
